# EUROPEAN PATENT SPECIFICATION

(11) **EP 2 116 184 B1**
(45) Date of publication and mention of the grant of the patent: **04.07.2012**
(21) Application number: 09006182.1
(22) Date of filing: 06.05.2009
(51) Int. Cl.: A61B 5/07, A61B 1/04

(54) **Antenna for capsule type medical device**
Antenne für medizinische Vorrichtung des Kapseltyps
Antenne pour dispositif médical de type capsule

(30) Priority: 07.05.2008 JP 2008121513
(43) Date of publication of application: 11.11.2009
(73) Proprietor: Olympus Medical Systems Corp., Tokyo 151-0072 (JP); Panasonic Corporation, Kadoma-shi Osaka 571-8501 (JP)
(72) Inventor: Takenaka, Tomoya, Oita-shi Oita 870-0126 (JP); Homan, Masatoshi, Tokyo 151-0072 (JP); Asakawa, Yasuteru, Osaka 540-6207 (JP); Kimura, Jun'ichi, Osaka 540-6207 (JP); Fukushima, Susumu, Osaka 540-6207 (JP); Kitagawa, Motoyoshi, Osaka 540-6207 (JP)
(74) Representative: von Hellfeld, Axel

(56) References cited:
- EP-A- 1 342 447
- EP-A- 1 702 553
- EP-A- 1 707 102
- EP-A- 1 749 472
- US-A1- 2006 241 422
- US-A1- 2006 258 901

## Description

### TECHNICAL FIELD

The present invention relates to an antenna used for a capsule type medical device.

### BACKGROUND ART

In recent years, the capsule type medical device that is inserted into the body of a subject and conducts intra-subject observation, examination, and medical cure or medical treatment has been in practical use. The capsule type medical device includes a capsule type casing, and internal components, such as an imaging unit, a transmitting unit, an antenna, or a power source, which are housed in the casing. As such a capsule type medical device, a capsule endoscope is known, and as the capsule endoscope, there is a swallowable capsule which is described, for example, in Japanese Patent Application Laid-Open No. 2005-329247.

In the aforementioned capsule type medical device, internal components are arranged in proximity to each other like one collective unit. Further, this collective unit of internal components is arranged inside the capsule type casing leaving little space between the unit and the casing. Therefore, even if an attempt to add an internal component in the capsule type casing, or to replace an existing component with another that requires additional space is made, in the current situation the capsule type casing has no extra space for the accommodation. Moreover, downsizing of the capsule type medical device is difficult.

US 2006/241422 discloses a device and method for in vivo imaging, for example, using an in vivo imaging device including a circuit board having rigid sections and flexible sections. The circuit board may include one or more layers and an antenna may be embedded into one or more layers.

EP 1 707 102 discloses a capsule-type medical apparatus having a plurality of comparatively rigid wiring board sections on which parts constituting a functional circuit are mounted, and a comparatively flexible wiring board section that connects the plurality of the rigid wiring board sections. Further, the flexible wiring board section is extended from a straight-line portion formed on the rigid wiring board section as well as the rigid wiring board section and the flexible wiring board section are housed in a sealed container by folding the flexible wiring board section so that the adjacent rigid wiring board sections oppose to each other. Consequently, housing efficiency of the sealed container improves so that insertion of the capsule-type medical apparatus into a subject body can be easily performed.

EP 1 342 447 discloses a capsule endoscope that includes means for illuminating an object, means for imaging the object, and a transparent cover having a center of curvature. The transparent cover covers the illumination means and the imaging means, and the imaging means includes an objective optical system and an image detecting element. The illumination means is positioned relative to the image detecting element, as viewed axially from the object side of the capsule endoscope, so that an area that is symmetrically positioned about the optical axis of the objective optical system from a light emitting area of the illumination means overlaps an area of the image detecting element, but does not overlap any areas of the image detecting element that are used for image detection.

### DISCLOSURE OF INVENTION

An antenna according to claim 1 is provided. The antenna is an antenna, for a capsule type medical device, to be embedded in the capsule type medical device. The antenna may include an antenna conductor, and a sheet-like antenna substrate that is closely attached to the sheet-like antenna conductor to at least partly electrically contact with the antenna conductor.

The above and other features, advantages and technical and industrial significance of this invention will be better understood by reading the following detailed description of presently preferred embodiments of the invention, when considered in connection with the accompanying drawings.

### BRIEF DESCRIPTION OF DRAWINGS

Fig. 1 is a longitudinal cross section of a capsule type medical device according to a first embodiment;
Fig. 2 is an A-A cross section of the capsule type medical device shown in Fig. 1;
Fig. 3 is a B-B cross section of the capsule type medical device shown in Fig. 1;
Fig. 4 is a developed view of a flexible substrate shown in Fig. 1;
Fig. 5A is a plan view of an antenna conductor shown in Fig. 1;
Fig. 5B is a side view of the antenna conductor shown in Fig. 5A;
Fig. 6 is a cross section of the main portion of the antenna conductor shown in Fig. 1;
Fig. 7 is a longitudinal cross section of a capsule type medical device according to a second embodiment;
Fig. 8 is a C-C cross section of the capsule type medical device shown in Fig. 7;
Fig. 9 is a D-D cross section of the capsule type medical device shown in Fig. 7;
Fig. 10 is a cross section of the main portion of an antenna conductor shown in Fig. 7;
Fig. 11A is a plan view of the antenna conductor shown in Fig. 7; and
Fig. 11B is a side view of the antenna conductor shown in Fig. 11A.

### BEST MODE(S) FOR CARRYING OUT THE INVENTION

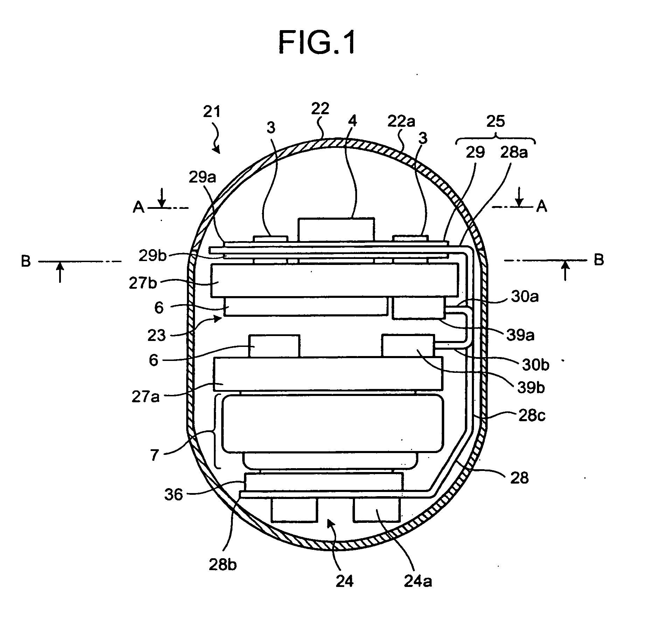

A capsule type medical device includes an antenna which is used to transmit information acquired by components of the capsule type medical device to outside, and to receive information given from outside. The inventors of the present invention focused their attentions on the fact that the conventional antenna housed in the capsule type medical device is cubic in shape, and therefore there exists dead space inside of the antenna as illustrated in Fig. 1 of Japanese Patent Application Laid-Open No. 2005-329247. The inventors of the present invention found that it is possible to provide more space by reducing or eliminating the dead space.

The present invention as outlined in claim 1 relates to an antenna embedded in the capsule type medical device. The antenna includes an antenna conductor, and a sheet-like antenna substrate that electrically contacts with at least a part of the antenna conductor the antenna conductor comprises a plurality of conductive convex portions provided on a side of the antenna conductor that is not in contact with the antenna substrate. The antenna conductor and the sheet-like antenna substrate are closely attached to each other. The antenna, in which antenna conductor and the sheet-like antenna substrate are closely attached to each other, like the antenna of the present invention, has an advantage of hardly yielding wasteful space in the antenna itself.

The antenna conductor and the antenna substrate, which are components of the antenna of the present invention, are described below in detail.

### --Antenna Conductor--

The material of the antenna conductor used in the present invention is not particularly limited, and known conductive materials may be used. For example, copper, copper alloy, silver, silver alloy, gold, gold alloy, tin alloy, or conductive adhesive may be used. Among copper alloys, copper-zinc alloy is preferable; among conductive adhesives, a conductive adhesive made by kneading silver into epoxy resin is preferable.

Shapes of the antenna conductor are not particularly limited, but either a wire-shaped or a sheet-like antenna conductor may be used. When the wired-shaped antenna conductor is employed, the wire diameter, wire length, or arranged shape thereof are not particularly limited, and can be appropriately decided, depending on the purpose. When the sheet-like antenna conductor is employed, the thickness, shape, and the like, of the antenna conductor are not particularly limited, and can be appropriately decided depending on the purpose. For example, a C-shaped antenna conductor can be used. With the C-shaped antenna conductor, a loop-antenna can be realized.

The shape of the antenna conductor can be decided depending on the shape of the antenna substrate, which is used in combination with the antenna conductor. For example, if an antenna substrate has a hollow, the antenna conductor is preferably shaped not to cover the hollow. Combinations of the antenna substrate and the antenna conductor are described later in detail in "Combination of Antenna Conductor and Antenna Substrate" section.

If the antenna conductor is sheet-like, one surface of the antenna conductor is preferably provided with conductive convex portions. It is possible to increase the surface area, and reduce the electrical resistivity of the antenna conductor by providing the conductive convex portions to the surface of the antenna conductor.

The number of convex portions are not particularly limited, and can be appropriately decided in accordance with purposes based on, for example, radiation characteristic, easiness of manufacturing, mechanical strength, and raw material cost.

The shape of the convex portion is not particularly limited, and may be appropriately decided in accordance with purposes based on, for example, radiation characteristic, easiness of manufacturing, mechanical strength, and raw material cost. When a plurality of convex portions is provided, all of the convex portions may be the same in shape, or a plurality of shapes may be adopted.

The positions of the convex portions to be arranged are not particularly limited, and can be appropriately decided in accordance with the purpose, based on, for example, radiation characteristic, easiness of manufacturing, mechanical strength, and raw material cost.

The material for forming the convex portions is not particularly limited, and can be appropriately decided in accordance with the purpose based on, for example, radiation characteristic, easiness of manufacturing, mechanical strength, and raw material cost. For example, the employed material can be at least one material selected from the group of, the same material with the material of the antenna conductor, solder, and conductive adhesive. Conventionally known materials can be used as the solder or the conductive adhesive.

Low-reflectivity processing may be performed on at least a part of the antenna conductor. The low-reflectivity processing can reduce the influence of light reflected by the antenna conductor on the result of imaging by the capsule type medical device. The low-reflectivity processing may be performed by, for example, making the surface of the antenna conductor rough, or providing the low reflective film on the surface of the antenna conductor. Material and thickness of the low reflective film are not particularly limited, and can be appropriately decided in accordance with the purpose.

### --Antenna Substrate--

The material of the antenna substrate is not particularly limited, and a conventionally known non-conductive material may be used. For example, glass epoxy board, paper phenol board, paper epoxy board, glass composite board, Teflon® board, alumina board, photo-solder resist board, liquid crystal polymer board, or polyimide board may be used.

The color of the antenna substrate is not particularly limited. Preferably, the antenna substrate is transmissive for visible light; more preferably, is colorless and transparent; and still more preferably, has 80% or more transmittance for visible light.

The shape of the antenna substrate is not particularly limited, but may be appropriately decided in accordance with the purpose. For example, the antenna substrate can be circular, oval, quadrangular, rectangular, polygonal, or curvilinearly polygonal in shape. The antenna substrate may be provided with a hollow. The hollow antenna substrate can be, for example, annular.

Low-reflectivity processing may be performed on at least a part of the antenna substrate. The low-reflectivity processing can reduce the influence of light reflected by the antenna substrate on the result of imaging by the capsule type medical device. The low-reflectivity processing may be performed by, for example, making the surface of the antenna substrate rough, or providing the low-reflective film on the surface of the antenna substrate. Material and thickness of the low reflective film are not particularly limited, and can be appropriately decided in accordance with the purpose.

As illustrated in Fig. 1, the antenna substrate may be linked to another substrate. One example of the other substrate is a transmitting substrate that holds a transmitter which is one of the components of the capsule type medical device. A link portion that links the antenna substrate with the other substrate is preferably flexible. As the link portion is flexible, the handling ability in installing the components of the capsule type medical device into the casing improves.

### --Combination of Antenna Conductor and Antenna Substrate--

The combination of the antenna conductor and the antenna substrate is not particularly limited. Although it is possible to appropriately combine the aforementioned antenna conductors and the antenna substrates, preferable examples are described below.

For example, a combination of the antenna substrate having a hollow and the antenna conductor shaped (i.e., formed) so as not to cover the hollow, or a combination of the antenna substrate having the hollow and the antenna conductor which is arranged so as not to cover the hollow are preferable. More preferably, the antenna substrate having the hollow is annular in shape. By adopting such a combination, an antenna having a hollow can be obtained. Then, by using such an antenna, for example, it is possible to arrange other components of the capsule type medical device in the hollow portion, or it is possible to arrange an imaging unit, which is an exemplary component of the capsule type medical device, at an internal side of the antenna substrate. The latter example is explained in more detail. In the conventional capsule medical device, it is impossible to arrange a component between an exterior package of the capsule type medical.device and the imaging unit in such a manner that the viewing field of the imaging unit is not blocked off. When the antenna has a hollow as in the present invention, however, unlike the conventional device, it is possible to arrange the antenna between the exterior package of the capsule type medical device and the imaging unit because the imaging unit can observe the outside through the hollow.

Another preferable example is a combination of the antenna substrate which is transmissive to visible light and the antenna conductor shaped in such a manner that the antenna conductor does not cover all the surfaces of the antenna substrate. Still another preferable example is a combination of the antenna substrate which is transmissive to visible light and the antenna conductor arranged in such a manner that the antenna conductor does not cover all the surfaces of the antenna substrate. Preferably, the antenna substrate is transparent and colorless; still preferably, the antenna substrate has 80% or more transmittance for visible light. By adopting such combinations, it is possible to obtain an antenna partly transmissive to the visible light. By adopting the antenna as described above, it is possible to arrange the imaging unit, which is one example of the components of the capsule type medical device, at an internal side of the antenna substrate. To explain more specifically, as described earlier, no component is arranged between the exterior package and the imaging unit in the conventional capsule type medical device. However, when the antenna has a visible-light-transmissive part as in the present invention, it is possible to observe the outside through the visible-light-transmissive part. Therefore, unlike the conventional device, it possible to arrange the antenna between the exterior package of the capsule type medical device and the imaging unit. The visible-light-transmissive antenna substrate may be provided with a hollow as described above.

The exemplary forms of the antenna according to the present invention are described below; however the present invention is not limited to the description below.

In Fig. 1 to Fig. 6, a conductor pattern of a link portion 28c is connected to a connecting terminal 31a at an upper side of an antenna substrate 28a. The connecting terminal 31a is formed at a position that corresponds to the vicinity of one end portion of an antenna conductor 29a. An intermediate terminal 32a is provided on a front side of the antenna substrate 28a, and at a position that corresponds to the vicinity of the other end portion of the antenna conductor 29a. A fixing land 33a, for preventing the antenna conductor 29a from uplifting, is provided on the front side of the antenna substrate 28a at a point that corresponds to an approximately intermediate point of the antenna substrate 29a. The fixing land 33a of the front side is not connected to other circuits, and is independent.

The intermediate terminal 32a is connected to an intermediate terminal 32b that is provided on a back side of the antenna substrate 28a via a throughhole 34. The intermediate terminal 32b is formed at a position that corresponds to the vicinity of one end portion of an antenna conductor 29b. A connecting terminal 31b, at the back side of the antenna substrate 28a, is formed at a position that corresponds to the other end portion of the antenna conductor 29b, and is connected to the conductor pattern of the link portion 28c. A fixing land 33b, for preventing the antenna conductor 29b from uplifting, is provided on the back side of the antenna substrate 28a at a point that corresponds to an approximately intermediate point of the antenna conductor 29b. The fixing land 33b of the front side is not connected to other circuits either, and is independent.

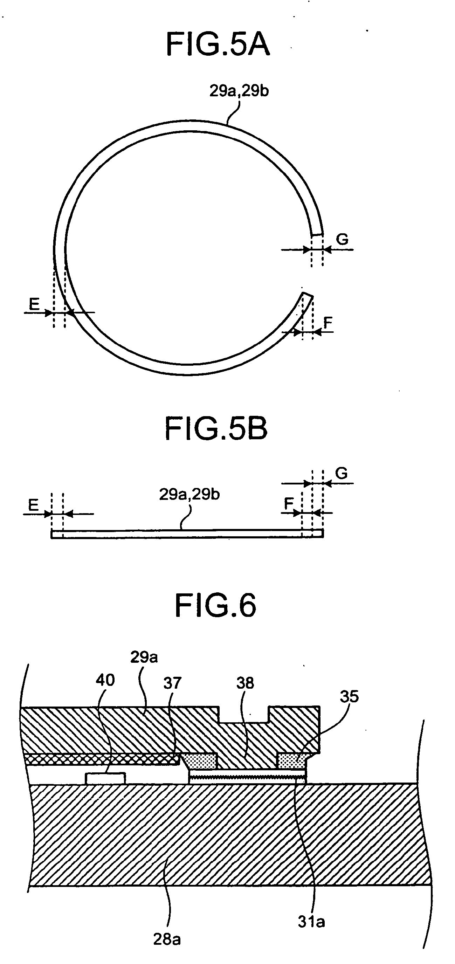

The shape of the antenna conductor 29a (29b) is schematically illustrated in Figs. 5A and 5B. Fig. 5A is a plan view of the antenna conductor 29a (29b), and Fig. 5B is a side view of the antenna conductor 29a (29b). In Figs. 5A and 5B, sections denoted by E, F, and G correspond with each other, respectively.

The antenna conductor 29a and the antenna conductor 29b are mounted to the front side and the back side of the antenna substrate 28a, respectively. The connecting terminal 31a and the antenna conductor 29a, the fixing land 33a and the antenna conductor 29a, the intermediate terminal 32a and the antenna conductor 29a, the intermediate terminal 32b and the antenna conductor 29b, the fixing land 33b and the antenna conductor 29b, and the connecting terminal 31b and the antenna conductor 29b, are respectively connected by means of soldering (not shown). Although soldering is used in the first embodiment, the conductive adhesive may be used instead.

Thus, the antenna conductors 29a and 29b are linked and form a spiral antenna conductor 29 extending between the connecting terminal 31a and the connecting terminal 31b, whereby a two-turn-wound helical antenna is formed on the antenna substrate 28a. Although in the first embodiment, the two-turn-wound helical antenna is formed, this is not a limiting example. For example, two loop antennas may be formed instead.

The antenna conductor 29a and the antenna conductor 29b, which are formed with thin sheets, are separately mounted on respective surfaces of the antenna substrate 28a. The antenna conductor 29a and the antenna conductor 29b are connected by means of soldering to form an antenna. Thus, a thin sheet-like antenna is formed. Therefore, further downsizing of a capsule type casing 22, and additions of new internal components or functions into the capsule type casing 22 of the same shape and the same size can be easily realized without compromising the imaging function of an imaging unit 4.

In the above configuration, a portion between the connecting terminal 31a and the connecting terminal 31b works as an antenna. Therefore, antennas of different lengths are easily obtained by appropriately changing positions of the connecting terminal 31a and the connecting terminal 31b, or the intermediate terminal 32a and intermediate terminal 32b, without changing the shapes of the antenna conductor 29a or the antenna conductor 29b.

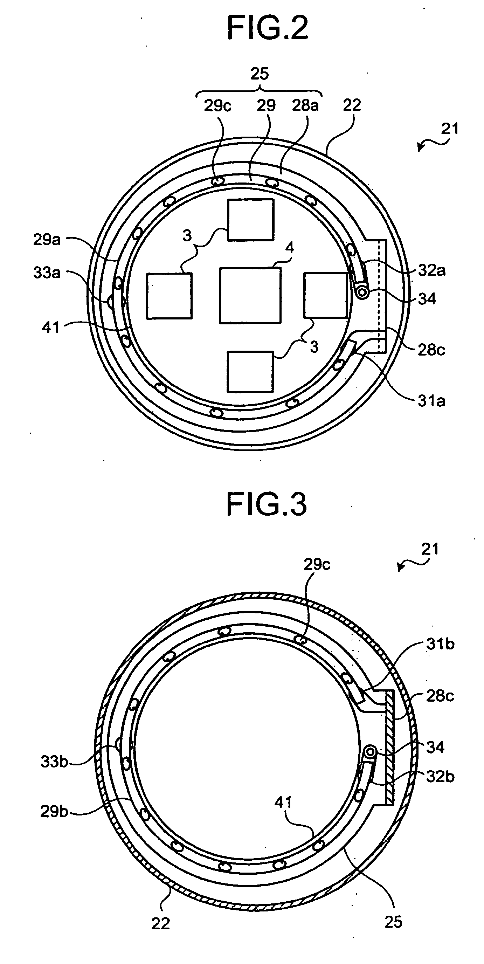

Moreover, in Fig. 2 and Fig. 3, if protruding portions 38 (see Fig. 6) that protrude toward the antenna substrate 28a are provided on each of the antenna conductor 29a and the antenna conductor 29b, at positions corresponding to the connecting terminals 31a and 31b, the intermediate terminals 32a and 32b, and the fixing lands 33a and 33b, the antenna conductor 29a and the antenna conductor 29b are elevated between the protruding portions 38. Therefore, it is possible to form other pattern (conductor 40 in Fig. 6) in the space formed between the antenna substrate 28a and the antenna conductors 29a and 29b.

A hole 41 is formed in the antenna substrate 28a, in a region surrounded by the antenna conductor 29. The hole 41 is an example of the hollow portion. As described above, a light emitting unit 3 and the imaging unit 4 are arranged in and penetrate through the hole 41. Being provided with the hole 41, the antenna substrate 28a hardly enters the imaging area of the imaging unit 4 even when the antenna substrate 28a is arranged in front of the imaging unit 4. Alternatively, a colorless, transparent member may be arranged at a portion corresponding to the hole 41, as far as the colorless, transparent member allows the imaging by the imaging unit 4. The transparent member can increase the strength of the antenna substrate 28a. Still alternatively, the entire antenna substrate 28a may be formed with a colorless, transparent material.

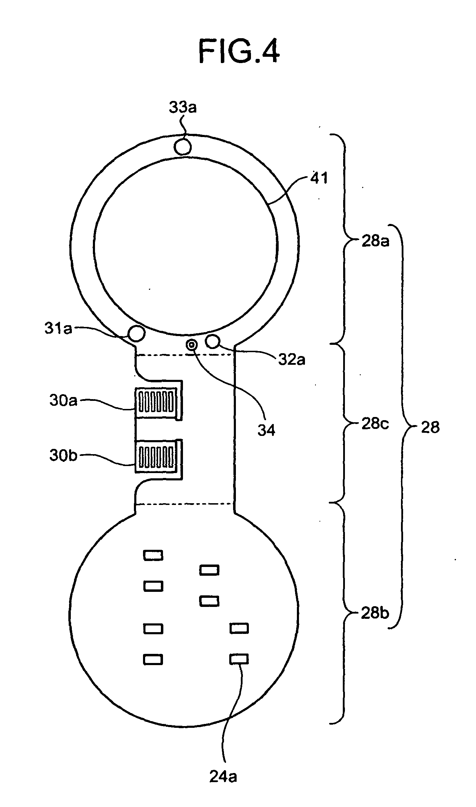

In the first embodiment which is not claimed, the size or the like of the hole 41 is set such that the light emitting unit 3 and the imaging unit 4 can be placed inside and pass through the hole 41 when the antenna substrate 28a in an extended state as illustrated in Fig. 4 is folded as illustrated in Fig. 1. In Fig. 2 and Fig. 3, the hole 41 is a single open-ended hole; however a separate hole 41 may be provided for each of the imaging unit 4 and the light emitting unit 3.

Next, a manufacturing method of a capsule type medical device 21 of the first embodiment according to the present invention is explained which does not form part of the claimed invention. First, creamy solder is applied on the connecting terminal 31a, fixing land 33a, and intermediate terminal 32a of the antenna substrate 28a by means of screen printing or the like, and the antenna conductor 29a is mounted onto the creamy solder. Subsequently, the antenna conductor 29a is connected and fixed to the antenna substrate 28a by melting the solder in a reflow furnace:

Next, creamy solder is applied on spots where the electronic parts are mounted, as well as on the connecting terminal 31b, fixing land 33b, and intermediate terminal 32b of the antenna substrate 28a, by means of screen printing and the like, and the antenna conductor 29b is mounted onto the creamy solder. Then, the electronic parts and antenna conductor 29b are connected and fixed to a flexible substrate 28 by melting the solder in the reflow furnace.

Subsequently, from the extended state as illustrated in Fig.4, the antenna substrate 28a and the link portion 28c are folded at predetermined positions, and then a connecting portion 30a and a connecting portion 30b are inserted into a connector 39a and a connector 39b respectively. Meanwhile, at this time, as illustrated in Fig. 1 to Fig. 3, the light emitting unit 3 and the imaging unit 4 are assembled so as to pass through the hole 41.

Then, a battery (hereinafter also referred to as "power source") 7 is interposed between a substrate 27a and a terminal substrate 36. However, to avoid unnecessary consumption of the battery 7 when not in use, the battery 7 is housed in the capsule type casing 22 in such a state that insulating paper is interposed between the battery 7 and the substrate 27a, and/or between the battery 7 and the terminal substrate 36. The insulating paper is removed when the capsule type medical device 21 is used.

The antenna conductor 29a and antenna conductor 29b, as described above, are formed by sheet processing of thin sheet-like metal, namely by punching out the thin sheet-like metal with a so called press work. Therefore, productivity of such process is very high, and the production cost of the antenna conductor 29a and antenna conductor 29b is low. In the first embodiment, as a brass sheet with a thickness of 0.15 mm is used for the antenna conductor 29a and antenna conductor 29b, antirust treatment is unnecessary because the brass hardly rusts. Although brass is used in the first embodiment, a copper sheet may be used instead. In this case, the antenna efficiency improves because the resistivity of the conductor can be reduced.

Moreover, in the first embodiment, as illustrated in Fig. 6, an insulating film 37 is formed on the antenna conductor 29a and the antenna conductor 29b, on a surface facing the antenna substrate 28a. The insulating film 37 is formed with resin having heat resistance such as polyimide and the like. In the antenna conductor 29a and antenna conductor 29b, the insulating film 37 is not formed on positions that correspond to the connecting terminal 31a, the connecting terminal 31b, the fixing land 33a, the fixing land 33b, the intermediate terminal 32a, and the intermediate terminal 32b. Thus, as illustrated in Fig. 6, it is possible to reduce fluctuations in the antenna length of the antenna conductor 29 because solder 35 exclusively sticks to the positions where the insulating film 37 is not formed.

Meanwhile, in the first embodiment, due to machining burr of the antenna conductor 29a and antenna conductor 29b generated by the press work, it is necessary to prevent short-circuit from occurring between the antenna conductor 29a and antenna conductor 29b, and the conductor 40, which is formed beneath the antenna conductor 29a and antenna conductor 29b. Therefore, when the antenna conductor 29a and antenna conductor 29b are mounted onto the antenna substrate 28a, the antenna conductor 29a and antenna conductor 29b are mounted in such a manner that the direction of the burr is away from the antenna substrate 28a, or an insulation film is also formed on the conductor 40.

Moreover, in the first embodiment, although the antenna conductor 29 is constituted with the antenna conductor 29a mounted on the front side, and the antenna conductor 29b mounted on the back side, of the antenna substrate 28a, in the case a shorter antenna serves the purpose, only one of the antenna conductor 29a and the antenna conductor 29b may be mounted to one side of the antenna substrate 28a. However, in this case, the intermediate terminal 32a. and intermediate terminal 32b are unnecessary, and the connecting terminal 31a and connecting terminal 31b are formed on the same surface. Meanwhile, in this case, in the state the antenna substrate 28a is folded, the surface on which the antenna conductor 29 is mounted faces an opposite side from the side of the electronic parts on a transmitting substrate 28b. This is because, as the electronic parts mounted on the transmitting substrate 28b and the antenna conductor 29a are mounted on the same side in a state before the substrate 28 is folded, the reflow heating is required only once. Therefore, the capsule type medical device 21 with an efficient productivity is realized.

Furthermore, in the first embodiment, the antenna is a balanced type antenna in which both the connecting terminal 31a and the connecting terminal 31b are connected to a transmitting circuit on the transmitting substrate 28b, however, if either one of the connecting terminal 31a and connecting terminal 31b is exclusively connected to the transmitting circuit, it is possible to make the antenna an unbalanced type antenna.

Alternatively, if a capacitative element is provided serially or parallel to the antenna conductor 29, it is possible to make the antenna a tuned-type antenna. In this case, if a variable-capacitance diode that is capable of varying the capacitance is used as the capacitative element, it becomes possible to obtain a tuned-type antenna which is capable of varying transceivable frequencies. Meanwhile, in such tuned-type antenna, it is preferable to arrange the capacitative element or variable-capacitance diode in the vicinity of the antenna conductor 29, by this, it is possible to reduce jumping of interfering signals into the pattern and the like which connect between the antenna conductor 29 and the capacitative element.

In the antenna according to the first embodiment, it is possible to mount the capacitative element in the vicinity of the antenna conductor 29, because the antenna is realized with the antenna conductor 29 mounted on the antenna substrate 28a. The structure according to the first embodiment is also suitable for the tuned-type antenna.

Furthermore, the upper side (front side) of the antenna substrate 28a of the first embodiment is made in a dark color. Thus, it is possible to make the reflected light hard to enter the imaging unit 4, because the light radiated from the light-emitting unit 3 becomes hard to be reflected by the antenna substrate 28a. In the first embodiment, as an example of the low reflective film, a film is formed by applying a coating of a black color resist on the surface of the antenna substrate 28a. The antenna substrate 28a is made to be transparent, if an antenna 25 is provided in such a manner that the antenna 25 covers the area in front of the imaging unit 4. This is for making the antenna substrate 28a not interrupt the light radiated from the light emitting unit 3, and not interrupt image pickup by the imaging unit 4.

In the first embodiment, it is possible to remarkably reduce the installation space of the antenna, because the antenna conductor 29, which electrically contacts with the sheet-like antenna substrate 28a, is formed in such a manner that the antenna conductor 29 is closely attached to the sheet-like antenna substrate 28a. Furthermore, the antenna substrate 28a and the antenna conductor 29 are installed in an unused area, without disturbing the functions of the imaging unit 4 and the light emitting unit 3. Therefore, further downsizing of the capsule type casing 22, and additions of new internal components or functions into the capsule type casing 22 of the same shape and the same size can be easily realized.

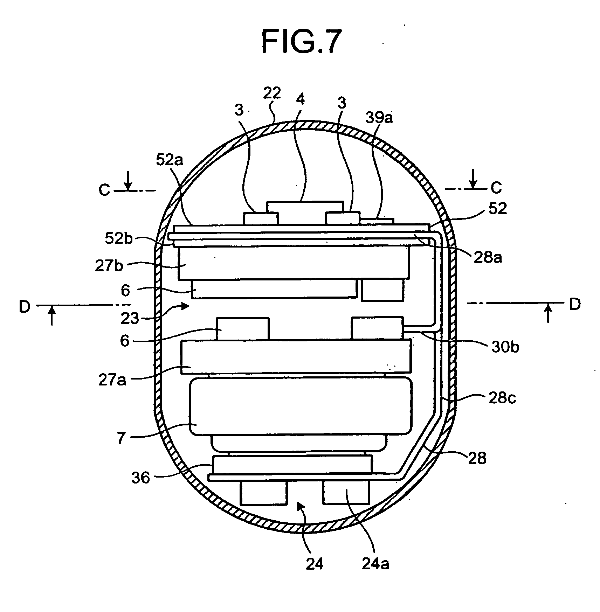

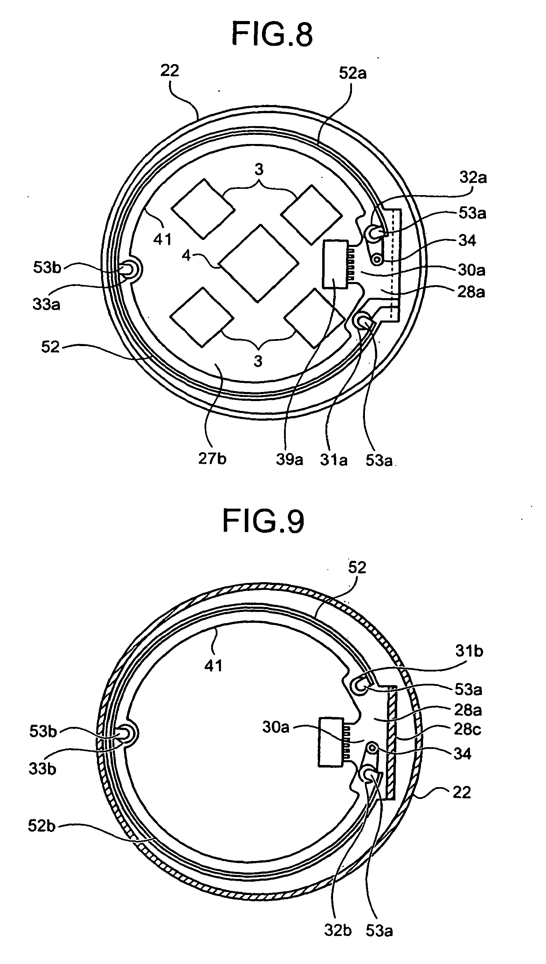

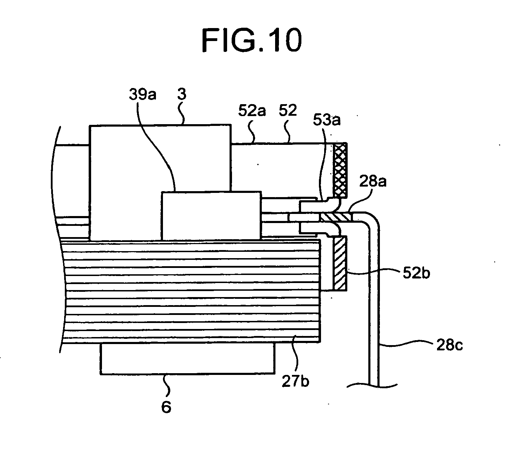

Subsequently, a second embodiment of the present invention is explained which has not been claimed. In the second embodiment, as illustrated in Fig. 7 to Fig. 11B, although being C-shaped like the antenna conductor 29, an antenna conductor 52, installed upright on the antenna substrate 28a, is used in place of the antenna conductor 29. More specifically, in the antenna conductor 52, a sheet-like antenna conductor 52a and a sheet-like antenna conductor 52b are mounted in such a state that the sheet-like antenna conductors 52a and 52b are upright against the antenna substrate 28a.

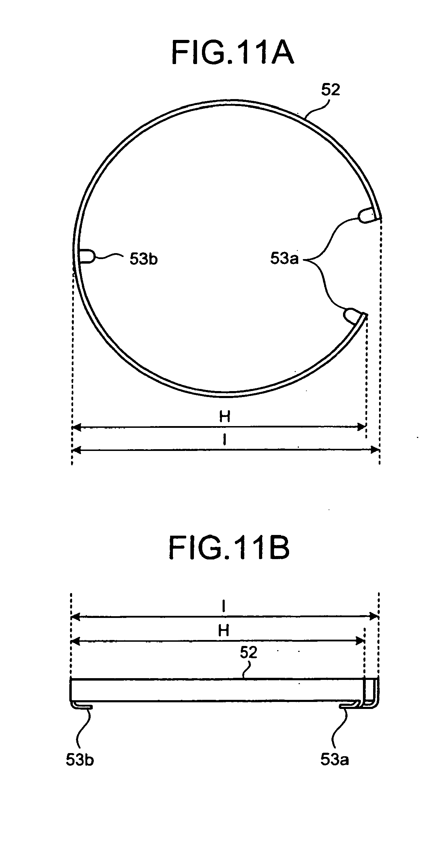

The shape of the antenna conductor 52a (52b) is schematically illustrated in Figs. 11A and 11B. Fig. 11A is a plan view of the antenna conductor 52a (52b), and Fig. 11B is a side view of the antenna conductor 52a (52b). In Figs. 11A and 11B, sections denoted by H and I correspond with each other, respectively.

A terminal section 53a and terminal section 53b respectively of the antenna conductor 52a and the antenna conductor 52b are bent substantially at a right angle with respect to the antenna conductor 52b. The terminal section 53a is provided at a position that corresponds to the connecting terminal 31a or the connecting terminal 31b, and the terminal section 53b is provided at a position that corresponds to the fixing land 33a or fixing land 33b. Although, the terminal section 53a and terminal section 53b are used as terminals to connect with the connecting terminal 31a, connecting terminal 31b, fixing land 33a, and the fixing land 33b, in addition, the terminal section 53a and terminal section 53b successfully exert similar functions to that of the protruding potions 38.

Then, the antenna conductor 52a and the antenna conductor 52b are subjected to curling process so as to have a C-shaped portion internal to the terminal section 53a and terminal section 53b. As in the first embodiment, a brass thin sheet with a thickness of 0.15 mm is preferably used for the antenna conductor 52a and antenna conductor 52b according to the second embodiment.

Then, the terminal section 53a is soldered to the connecting terminal 31a or connecting terminal 31b, and the terminal section 53b is soldered to the fixing land 33a and fixing land 33b. In this case, the solder 35 adheres to the terminal section 53a and terminal section 53b. Therefore, the solder 35 hardly adheres to other areas than the specified areas, and impedance of the antenna conductor 52 hardly varies.

Here, in the second embodiment, a diameter of a terminal substrate 27b is smaller than a diameter of the antenna conductor 52b. A side surface of the terminal substrate 27b is arranged to face the antenna conductor 52b. Namely, the antenna conductor 52b is arranged in such a manner that it surrounds the terminal substrate 27b. Thus, it is possible to lower the height of the antenna conductor 52 in the front side of the terminal substrate 27b. Therefore, it is possible to make it hard for the antenna conductor 52 to enter the imaging area of the imaging unit 4. Furthermore, in the terminal substrate 27b, it is possible to reduce the area, which faces the antenna substrate 28a, of the terminal substrate 27b; it becomes possible to mount more electronic parts on the front side of the terminal substrate 27b.

Meanwhile, the antenna conductor 52b may be mounted close to the outer circumference of the antenna substrate 28a as far as possible so that an upper surface of the antenna conductor 52b may face the front surface of the terminal substrate 27b. According to such a configuration, it is possible to effectively utilize, as an antenna area, the area about 0.5 mm from the outer circumference of the terminal substrate 27b. To this area, mounting the parts is practically impossible in general, in the general purpose device to which the electronic parts are mounted. According to the above configuration, it is possible to efficiently utilize the area of the flexible substrate 28.

Moreover, in the second embodiment, the connecting portion 30a is provided on the antenna substrate 28a, thus it becomes unnecessary to bend the connecting portion 30a separately from the antenna substrate 28a.

In the aforementioned first and second embodiments, although the antenna substrate 28a is arranged on the terminal substrate 27b, the antenna substrate 28a may be arranged between the substrate 27a and the terminal substrate 27b of an imager 23, or between the imager 23 and the battery 7, or between the imager 23 and a transmitting unit 24.

Moreover, in the aforementioned first and second embodiments, thicknesses of the antenna conductor 29a, the antenna conductor 29b, the antenna conductor 52a, and the antenna conductor 52b, which constitute the antenna conductor 29 and the antenna conductor 52, may be thickened by solder provided on the surface of the antenna conductors. According to the above, it is possible to reduce the electric resistive component and heighten the Q-value of the antenna conductor 29a, the antenna conductor 29b, the antenna conductor 52a, and the antenna conductor 52b. For example, as illustrated in Fig. 2 and Fig. 3, convex portions 29c may be partly built up with solder on the front side surfaces of the antenna conductor 29a and the antenna conductor 29b. The convex portions 29c may be formed on the whole surface of the antenna conductor 29a and the antenna conductor 29b. When the solder is applied to the antenna conductor 52a and the antenna conductor 52b, the solder spreads in a plane direction of the antenna substrate 28a so as to form a convex portion, and as a result, the sheet-like antenna conductor 52a, and the antenna conductor 52b become thick conductors. The convex portion may be formed with the conductive adhesive in place of the solder.

The capsule type medical device not part of the invention that can apply the antenna of the present invention is explained below referring to accompanying drawings.

Fig. 1 is a longitudinal cross section of the capsule type medical device 21 which applies the antenna of the present invention. According to the capsule type medical device 21 illustrated in Fig. 1, the light emitting unit 3, the imaging unit 4, the transmitting unit 24, the antenna 25, a control unit 6 that controls these units, and the power source (battery) 7 that supplies power to these units are housed in the capsule type casing 22. The light emitting unit 3 emits illuminating light inside the body when the image is taken. The imaging unit 4 takes images of inside of the body. The transmitting unit 24 is for at least transmitting information obtained inside the body of a subject to the outside. However, the components of the capsule type medical device of the present invention are not limited to aforementioned units. For example, the capsule type medical device without the light emitting unit is possible depending on the function of the imaging unit. The antenna, which is applied to the capsule type medical device 21 illustrated in Fig. 1, is formed by combining the antenna substrate 28a having the hollow with the antenna conductor 29 which is shaped in such a way not to cover the hollow. The light emitting unit 3 and the imaging unit 4, which are components of the capsule type medical device 21, are arranged in the hollow. Each of the components is explained in detail below.

### --Casing--

The capsule type casing 22, at least, plays a role of protecting the internal components of the capsule type medical device 21. The shape of the capsule type casing 22 is not particularly limited. For example, as illustrated in Fig. 1, the capsule type casing 22 may have a cylindrical shape, having dome shapes at both ends. If any of the internal components of the capsule type medical device 21 do not function properly when getting wet, the capsule type casing 22 may preferably be airtight.

Material for forming the capsule type casing 22 is not limited. For example, a plastic can be used.

When the imaging unit 4, which acquires the intra-subject information, has a function to acquire the optical information, the capsule type casing 22 may preferably have an optically transparent portion. For example, as illustrated in Fig. 1, the portions that form hemispherical domes may be formed with a transparent material 22a.

### --Imaging unit--

The imaging unit 4 has a function to acquire information from the subject. In Fig. 1, although the imaging unit 4 is arranged in such a manner to pass through the hollow of the antenna 25, if the imaging unit 4 is used in combination with the antenna 25 having the hollow, the imaging unit 4 may be arranged at a position from which the outside of the casing can be observed through the hollow.

### --Transmitting unit--

The transmitting unit 24 includes the transmitting substrate 28b and electronic parts 24a that are arranged on the transmitting substrate 28b. Moreover, as illustrated in Fig. 1, the transmitting substrate 28b may be linked to the antenna substrate 28a. Fig. 4 is an exemplary developed view of the linked transmitting substrate 28b and the antenna substrate 28a.

In Fig. 4, the antenna substrate 28a provided with the antenna 25, the transmitting substrate 28b provided with the transmitting unit 24, and the link portion 28c that links the transmitting substrate 28b and the antenna substrate 28a, are illustrated. The antenna substrate 28a and the transmitting substrate 28b can be made to face with each other by bending the link portion 28c. For example, in Fig. 1, the antenna substrate 28a is arranged above the upper surface of the terminal substrate 27b in such a manner that the antenna substrate 28a faces the terminal substrate 27b. On the other hand, the transmitting substrate 28b is arranged at a lower surface of the battery 7 in such a manner that the transmitting substrate 28b faces the substrate 27a. In this state, the transmitting substrate 28b is bent in such a manner that the electronic parts 24a face downward and the terminal substrate 36 faces upward. However, the present invention is not limited to this arrangement.

The link portion 28c is preferably flexible. The material which constitutes the link portion is not particularly limited; it can be appropriately decided based on the purpose.

When the transmitting substrate 28b and the antenna substrate 28a are to be linked, the transmitting unit 24 and the antenna conductor 29 can be electrically connected by forming the conductor pattern across the transmitting substrate 28b and the antenna substrate 28a, for example.

### --Power source--

The power source 7 at least plays the role to supply electric power to each of the components of the capsule type medical device 21. The power source is not particularly limited, but conventionally known batteries can be used.

### --Other components--

The capsule type medical device of the present invention can include components of the conventionally known capsule type medical device other than components described above, depending on the purposes.

In the example described above, although the capsule type medical device having a single imaging unit is explained, the present invention is not limited to the described example. The present invention can be applied to capsule type medical devices having a plurality of imaging units.

Furthermore, in the example described above, the antenna substrate 28a and the antenna conductors 29 and 52 are arranged in the vicinity of the imaging unit 4, by connecting the transmitting unit 24 with the antenna substrate 28a and antenna conductor 29, 52, using the flexible substrate 28 which has the link portion 28c. However, the antenna substrate 28a and antenna conductor 29, 52 may be arranged at an arbitrary positions in the capsule type casing 22. Even in this case, as the antenna substrate 28a and antenna conductor 29, 52 per se are downsized, it is possible to secure space for further storage in the capsule type casing 22.

In the antenna for the capsule type medical device according to the embodiments, it is possible to reduce or eliminate the dead space inside the antenna.

Moreover, in the capsule type medical device that includes the antenna for the capsule type medical device, it is possible to provide more space.

A capsule type medical device according to one embodiment includes a casing which is at least partly transparent, an imaging unit that is housed in the casing, a transmitting unit that is housed in the casing, a power source that is housed in the casing, and an antenna, for a capsule type medical device, housed in the casing. The antenna includes an antenna conductor, and a sheet-like antenna substrate which at least partly electrically contacts with the antenna conductor. The antenna substrate is colorless and transparent. The sheet-like antenna substrate is closely attached to the antenna conductor. The antenna conductor is arranged so as not to cover all part of the antenna substrate, or is formed so as not to cover all part of the antenna substrate. The imaging unit is arranged in a position from which outside of the casing can be observed through the antenna.

A capsule type medical device according to another embodiment includes a casing that is at least partly transparent, an imaging unit that is housed in the casing, a transmitting unit that is housed in the casing, a power source that is housed in the casing; and an antenna, for a capsule type medical device, housed in the casing. The antenna includes an antenna conductor, and a sheet-like antenna substrate which at least partly electrically contacts with the antenna conductor. The antenna substrate is closely attached to the antenna conductor. The antenna substrate is annular in shape and has a hollow. The antenna conductor is arranged so as not to cover the hollow, or is formed so as not to cover the hollow. The imaging unit is arranged in the hollow.

A capsule type medical device according to still another embodiment includes a casing which is at least partly transparent, an imaging unit that is housed in the casing, a transmitting unit that is housed in the casing, a power source that is housed in the casing, and an antenna, for a capsule type medical device, housed in the casing. The antenna includes an antenna conductor, and a sheet-like antenna substrate which at least partly electrically contacts with the antenna conductor. The antenna substrate is closely attached to the antenna conductor. The antenna substrate is annular in shape and has a hollow. The antenna conductor is arranged so as not to cover the hollow, or is formed so as not to cover the hollow. The imaging unit is arranged in a position from which outside of the casing can be observed through the hollow.

A capsule type medical device according to still another embodiment includes a casing which is at least partly transparent, an imaging unit that is housed in the casing, a transmitting unit that is housed in the casing, a power source that is housed in the casing, and an antenna, for a capsule type medical device, housed in the casing. The antenna is arranged out of a range of viewing field of the imaging unit. The antenna includes an antenna conductor, and a sheet-like antenna substrate which at least partly electrically contacts with the antenna conductor. The antenna substrate is closely attached to the antenna conductor.

A capsule type medical device according to still another embodiment includes a casing which is at least partly transparent, an imaging unit that is housed in the casing, a transmitting unit that is housed in the casing, a power source that is housed in the casing, and an antenna, for a capsule type medical device, housed in the casing. The antenna includes an antenna conductor, and a sheet-like antenna substrate which at least partly electrically contacts with the antenna conductor. The antenna substrate is closely attached to the antenna conductor. The transmitting unit includes a transmitting substrate and electronic parts that are arranged on the transmitting substrate. The antenna substrate and the transmitting substrate are linked, and the antenna substrate and the transmitting substrate are folded to face each other.

## Claims

1. An antenna (25), for a capsule type medical device (21), to be embedded in the capsule type medical device, the antenna comprising:
a sheet-like antenna conductor (29a;29b;29;52a;52b;52); and
a sheet-like antenna substrate (28a) that is closely attached to the sheet-like antenna conductor (29a;29b;29;52a;52b;52) to at least partly electrically contact with the antenna conductor (29a;29b;29;52a;52b;52), the antenna conductor (29a;29b;52a;52b) being arranged to overlap with at least one side of the antenna substrate (28a);
**characterized in that** the antenna conductor (29a; 29b; 52a; 52b) comprises:
a plurality of conductive convex portions (29c) provided on a side of the antenna conductor (29a;29b) that is not in contact with the antenna substrate (28a).

2. The antenna (25) according to claim 1, wherein
the convex portions (29c) are made of solder.

3. The antenna (25) according to claim 1, wherein
the convex portions are made of conductive adhesive.

4. The antenna (25) according to claim 1, wherein
the antenna substrate (28a) is provided with a throughhole (34), and the antenna conductor (29a;29b;29;52a;52b;52) is C-shaped, and
the antenna conductor (29a,29b;29;52a,52b;52) includes,
a first antenna conductor (29a;52a) having a first terminal and arranged on a first surface of the antenna substrate (28a), and
a second antenna conductor (29b;52b) having a second terminal and arranged on a second surface of the antenna substrate (28a), the first terminal and the second terminal being electrically connected with each other via the throughhole (34).

5. The antenna (25) according to claim 1, wherein
a surface of the antenna conductor (29a;29b;29;52a;52b;52) is at least partly provided with a low reflective film.

6. The antenna (25) according to claim 1, wherein
at least one surface of the antenna substrate (28a) is provided with a low reflective film.

7. The antenna (25) according to claim 1, wherein
the antenna substrate (28a) is transparent and colorless, and
the antenna conductor (29a;29b;29;52a;52b;52) is arranged so as not to cover all part of the antenna substrate (28a), or is formed so as not to cover all part of the antenna substrate (28a).

8. The antenna (25) according to claim 1, wherein
the antenna substrate (28a) is annular in shape and has a hollow in its center, and
the antenna conductor (29a;29b;29;52a;52b;52) is arranged so as not to cover the hollow, or is formed so as not to cover the hollow.

## Patentansprüche

1. Antenne (25) für eine medizinische Vorrichtung eines Kapseltyps (21) zum Einbetten in der medizinischen Vorrichtung des Kapseltyps, wobei die Antenne umfasst:
einen folienartigen Antennenleiter (29a; 29b; 29; 52a; 52b; 52) und
ein folienartiges Antennensubstrat (28a), das eng mit dem folienartigen Antennenleiter (29a; 29b; 29; 52a; 52b; 52) verbunden ist, um zumindest teilweise in elektrischem Kontakt mit dem Antennenleiter (29a; 29b; 29; 52a; 52b; 52) zu stehen, wobei der Antennenleiter (29a; 29b; 52a; 52b) so angeordnet ist, dass er mindestens eine Seite des Antennensubstrats (28a) überlappt;
**dadurch gekennzeichnet, dass** der Antennenleiter (29a; 29b; 52a; 52b) aufweist:
eine Vielzahl von leitenden konvexen Bereichen (29c), die auf einer Seite des Antennenleiters (29a; 29b), die nicht mit dem Antennensubstrat (28a) in Kontakt steht, vorgesehen sind.

2. Antenne (25) nach Anspruch 1, wobei
die konvexen Bereiche (29c) aus Lötmittel gebildet sind.

3. Antenne (25) nach Anspruch 1, wobei
die konvexen Bereiche aus leitendem Kleber gebildet sind.

4. Antenne (25) nach Anspruch 1, wobei
das Antennensubstrat (28a) mit einem Durchgangsloch (34) versehen ist und der Antennenleiter (29a; 29b; 29; 52a; 52b; 52) C-förmig ausgebildet ist, und
der Antennenleiter (29a; 29b; 29; 52a; 52b; 52) aufweist:
einen ersten Antennenleiter (29a; 52a), der einen ersten Anschluss besitzt und auf einer ersten Fläche des Antennensubstrats (28a) angeordnet ist, und
einen zweiten Antennenleiter (29b; 52b), der einen zweiten Anschluss besitzt und auf einer zweiten Fläche des Antennensubstrats (28a) angeordnet ist, wobei der erste und der zweite Anschluss über das Durchgangsloch (34) elektrisch miteinander verbunden sind.

5. Antenne (25) nach Anspruch 1, wobei
eine Fläche des Antennenleiters (29a; 29b; 29; 52a; 52b; 52) zumindest teilweise mit einem reflexionsarmen Film versehen ist.

6. Antenne (25) nach Anspruch 1, wobei
mindestens eine Fläche des Antennensubstrats (28a) mit einem reflexionsarmen Film versehen ist.

7. Antenne (25) nach Anspruch 1, wobei
das Antennensubstrat (28a) durchsichtig und farblos ist, und der Antennenleiter (29a; 29b; 29; 52a; 52b; 52) so angeordnet ist, dass er nicht den gesamten Teil des Antennensubstrats (28a) abdeckt, oder so ausgebildet ist, dass er nicht den gesamten Teil des Antennensubstrats (28a) abdeckt.

8. Antenne (25) nach Anspruch 1, wobei
das Antennensubstrat (28a) ringförmig ausgebildet ist und eine Aussparung in seiner Mitte aufweist, und
der Antennenleiter (29a; 29b; 29; 52a; 52b; 52) so angeordnet ist, dass er nicht die Aussparung abdeckt, oder so ausgebildet ist, dass er nicht die Aussparung abdeckt.

## Revendications

1. Antenne (25), pour un dispositif médical de type capsule (21), destinée à être incorporée dans le dispositif médical de type capsule, l'antenne comprenant :
un conducteur d'antenne de type feuille (29a ; 29b ; 29 ; 52a ; 52b ; 52) ; et
un substrat d'antenne de type feuille (28a) qui est étroitement fixé au conducteur d'antenne de type feuille (29a ; 29b ; 29 ; 52a ; 52b ; 52) pour un contact électrique au moins partiel avec le conducteur d'antenne (29a ; 29b ; 29 ; 52a ; 52b ; 52), le conducteur d'antenne (29a ; 29b ; 52a ; 52b) étant agencé de manière à se chevaucher avec au moins un côté du substrat d'antenne (28a) ;
**caractérisé en ce que** le conducteur d'antenne (29a ; 29b ; 52a ; 52b) comprend :
une pluralité de parties convexes (29c) conductrices prévues sur un côté du conducteur d'antenne (29a ; 29b) qui n'est pas en contact avec le substrat d'antenne (28a).

2. Antenne (25) selon la revendication 1, dans laquelle les parties convexes (29c) sont constituées d'une soudure.

3. Antenne (25) selon la revendication 1, dans laquelle les parties convexes sont constituées d'un adhésif conducteur.

4. Antenne (25) selon la revendication 1, dans laquelle
le substrat d'antenne (28a) est prévu avec un trou traversant (34), et le conducteur d'antenne (29a ; 29b ; 29 ; 52a ; 52b ; 52) est en forme de C, et
le conducteur d'antenne (29a ; 29b ; 29 ; 52a ; 52b ; 52) inclut
un premier conducteur d'antenne (29a ; 52a) ayant une première borne et agencé sur une première surface du substrat d'antenne (28a), et
un deuxième conducteur d'antenne (29b ; 52b) ayant une deuxième borne et agencé sur une deuxième surface du substrat d'antenne (28a), la première borne et la deuxième borne étant électriquement connectées l'une avec l'autre par l'intermédiaire du trou traversant (34).

5. Antenne (25) selon la revendication 1, dans laquelle
une surface du conducteur d'antenne (29a ; 29b ; 29 ; 52a ; 52b ; 52) est au moins partiellement prévue avec un film faiblement réfléchissant.

6. Antenne (25) selon la revendication 1, dans laquelle
au moins une surface du substrat d'antenne (28a) est prévue avec un film faiblement réfléchissant.

7. Antenne (25) selon la revendication 1, dans laquelle
le substrat d'antenne (28a) est transparent et incolore, et
le conducteur d'antenne (29a ; 29b ; 29 ; 52a ; 52b ; 52) est agencé de manière à ne pas recouvrir la totalité du substrat d'antenne (28a), ou est formé de manière à ne pas recouvrir la totalité du substrat d'antenne (28a).

8. Antenne (25) selon la revendication 1, dans laquelle
le substrat d'antenne (28a) est annulaire de forme et a un creux en son centre, et
le conducteur d'antenne (29a ; 29b ; 29 ; 52a ; 52b ; 52) est agencé de manière à ne pas recouvrir le creux, ou est formé de manière à ne pas recouvrir le creux.
